# EUROPEAN PATENT APPLICATION

(11) **EP 4 545 010 A1**
(43) Date of publication of application: **30.04.2025**
(21) Application number: 23214961.7
(22) Date of filing: 07.12.2023
(51) Int. Cl.: A61B 6/03, A61B 6/04, A61B 6/40, A61B 6/46, A61B 6/00, A61B 6/50

(54) **CT IMAGING METHOD**

(30) Priority: 24.10.2023 US 202363545427 P
(71) Applicant: Koninklijke Philips N.V., 5656 AG Eindhoven (NL)
(72) Inventor: GRASS, Michael, Eindhoven (NL); KABUS, Sven, Eindhoven (NL); KOEHLER, Thomas, 5656AG Eindhoven (NL); WILD, Sebastian, Eindhoven (NL); VEMBAR, Manindranath, Eindhoven (NL)
(74) Representative: Philips Intellectual Property & Standards

(57) **Abstract**

An image generation and display method for use in CT imaging, for example cone beam sequential (step-and-shoot) imaging. The method utilizes the overlap between axially neighboring projection datasets acquired in a sequential imaging procedure to generate a first and second image representation of a region, where the first image is formed by stitching together neighboring image volumes aiming to form a continuous image representation of the anatomy, and the second image is formed as a modified version of the first, wherein a portion of the overlap region between two neighboring image volumes is rendered using data from a single one only of the two overlapping volumes. This allows for an anatomical structure which extends over the overlap region to be visualized more accurately, free from any potential artefacts occurring at the boundary between neighboring volumes in the rendered image.

## Description

### FIELD OF THE INVENTION

The present invention relates to the field of computed tomography imaging, and in particular to CT image reconstruction.

### BACKGROUND OF THE INVENTION

Within the field of CT imaging, there are different possible CT scanning modes.

One mode of CT scan is helical imaging. In this mode, the X-ray source, carried by a rotatable gantry, continuously rotates around a patient while the patient is simultaneously and continuously moved through the gantry. This creates a spiral or helical path of data acquisition. Helical scanning can achieve faster scan times. In some applications, it may reduce total radiation dose. It also allows for imaging dynamic phenomena such as blood flow, due to its continuous nature.

A different mode of CT scan is sequential CT scanning (otherwise known as step-and-shoot or axial scanning). In sequential CT scanning, a patient is moved incrementally through the gantry (along the axial direction). At each increment or "step", the X-ray source and detector rotate around the patient, capturing a series of projections at various angles. Data for a complete rotation may be collected for each axial position, or for just a portion of a rotation, or for more than a single rotation. After the whole or partial rotation, the patient is moved, and the process is repeated. Each whole or partial rotation produces data for one "slab" (containing a couple of axial images, i.e. slices) of the body. After the projection data has been acquired in this way, typically, each slice may be reconstructed using image reconstruction algorithm such as filtered back projection (FBP). The sequence of slices may then be combined into a single 3D (volumetric) image of the overall imaging zone covered by the scan.

Sequential CT scanning may achieve higher image resolution compared with helical imaging. In addition, in some cases it may achieve a lower dose than helical imaging. In particular, for imaging cyclically moving anatomical structures, such as the heart, lungs, liver etc., it may be desired to obtain images of the structure only during a specific phase of the cyclical motion. In this event, a suitably gated sequential scanning operation can obtain the images at each gantry position with a lower dose compared with a helical scan.

With regards to gated imaging, this enables obtaining an image of a slice or volumetric region of the imaging zone which corresponds to only a single point or period during a movement cycle (e.g. a single point in a cardiac cycle). Gating can be performed in two ways: prospective gating and retrospective gating.

In prospective gating, before the scan, a specific point in the cardiac cycle is selected (often during diastole when the heart is relatively motionless). The CT scanner is controlled to acquire images only at that pre-selected point in the cardiac cycle for each table position or 'step', for example triggered or guided using an ECG sensor attached to the patient. This reduces radiation exposure since the scanner needs only administer radiation during the small portion of the cardiac cycle. In retrospective gating, the CT scanner continuously acquires images throughout the entire cardiac cycle. Data from all phases of the cardiac cycle are stored. After the scan, a target phase for image reconstruction is retrospectively chosen, and imaging data outside of that phase is discarded for purposes of image reconstruction. This method involves a relatively higher radiation dose as the scanner is continuously imaging.

To perform a prospective gating method, sequential scanning is typically the more appropriate method since the same relatively brief acquisition period, synchronized to the heart cycle, can be obtained for each individual axial slice. The slices may then be combined to form an overall 3D image of a region.

Cone Beam Computed Tomography (CBCT) employs a divergent X-ray beam, e.g. shaped like a cone, allowing a single rotation to capture data spanning a volumetric cylinder, rather than only a single slice. This allows for faster imaging.

Cone beam imaging can employ both helical and step-and-shoot scan modes.

In a step-and-shoot mode, distinct volumetric datasets are acquired sequentially at each of a series of axial positions by moving the patient or source-detector system incrementally along the axial direction.

The imaging procedure in this case may include the following steps. For each axial position of the patient relative to the source-detector system, the X-ray source and detector revolve around the patient, capturing a projection dataset at multiple angles. After one full (or partial) rotation at the respective axial location, the source and detector are moved to a next axial position relative to the patient, and the process repeats. The scan data acquired at each axial position produces a volumetric dataset. With regards to image reconstruction, for cone beam data, the most common reconstruction algorithm is the Feldkamp-Davis-Kress (FDK) algorithm. This algorithm corrects the divergent nature of the X-ray beam in CBCT by rebinning the data into a parallel or fan-beam format, then applying backprojection. Alternatively, iterative reconstruction methods can be used. These methods estimate the image and refine the reconstruction iteratively, offering potentially better image quality and reduced artifacts.

Since each acquisition in the step-and-shoot process yields a volumetric dataset, these datasets need to be combined to produce a comprehensive view. This combination is called stitching.

Optionally, datasets are registered and aligned based on overlapping regions before stitching with the aim to ensure a smooth transition between datasets. Stitching algorithms may employ weighting functions to account for redundant data in the overlapping regions and reduce artifacts.

However, step artifacts in cardiac CT, particularly in step-and-shoot acquisition modes, can arise at the boundaries between stitched adjacent axial volumetric datasets.

For example, in cardiac CT imaging, one potential source of step artefacts is heart rate variability. If there is variability in the patient's heart rate between subsequent axial acquisitions, this may cause misalignment or inconsistency between adjacent datasets. Another potential source of step artefacts is respiratory motion: Even if patients are asked to hold their breath during scanning, there can be subtle variations in the position of the heart due to respiratory motion. If these shifts occur between two datasets, it can cause noticeable step artifacts at the stitching boundaries. The visualization of the cardiac anatomy may hence show discontinuities which make it difficult to interpret the image volume and more specifically to assess the status of the coronary arteries. Even in cases where no explicit displacement is visible, a small discontinuity may be visible in the image which raises doubts that coronary artery lesions may be analyzed with sufficient accuracy. Thus, the interpretation of coronary artery lesions may be compromised at the border between sub-volumes of the step-and-shoot cardiac CT data sets.

### SUMMARY OF THE INVENTION

The invention is defined by the claims.

According to examples in accordance with an aspect of the invention, there is provided a method for visualization of CT scan data acquired in a sequential CT scanning protocol (otherwise known as a step-and-shoot scan protocol).

The method comprises receiving a plurality of CT volumetric datasets, each corresponding to a different axial acquisition position along the sequential CT scanning protocol, wherein axially neighboring volumetric datasets partially overlap in the axial direction across a respective overlap volume.

The method may further comprise generating a first image from the plurality of CT volumetric datasets, the first image including at least a portion of at least one of the overlap volumes, the at least one overlap volume representing an overlap between a first and a second of the CT volumetric datasets.

The method may further comprise rendering and displaying a representation of the first image using a user interface.

The method may further comprise receiving an indication of a sub-region of the first image coinciding at least partially with said at least one overlap region.

The method may further comprise generating a second image from the plurality of CT volumetric datasets in which the sub-region is rendered based on only said first CT volumetric dataset, only said second CT volumetric dataset, or based on a transparency overlay of both the first and second CT volume datasets.

The method may further comprise rendering and displaying the second image using the user interface.

There is thus provided a method for visualization of step-and-shoot CT image data which permits a selected sub-region of the image overlapping with a boundary line or overlapping boundary region (e.g. transition region) between two neighboring axial volumes to be rendered to show a continuous representation of a portion of the first sub-volume which coincides with said sub-region, and/or a portion of the second sub-volume which coincides with said sub-region. This means that in a case where a step artefact occurs in a representation of an anatomically significant area within the aforementioned overlap or transition region, a new rendering can be generated in which the anatomically significant area is presented using just one or other of the two overlapping axial volume datasets, so that the step artefact no longer appears. This comes at the cost in the second image that there may be a new discontinuity created in the second image at the boundary of the selected sub-region. However, for purposes of examining an anatomically significant structure located in the selected sub-region, this new discontinuity is less important. Preferably, means may be provided to enable a user to switch alternately between the first image and the second image.

For example, in some embodiments, the method comprises controlling the user interface to present a user control permitting a user to select between display of the first image and display of the second image, e.g. to toggle between the first image and the second image.

In some embodiments, the receiving the indication of the sub-region comprises receiving a user input from the user interface indicative of a user-indicated region in the first image coinciding at least partially with said at least one overlap region. In other words, the sub-region is chosen by the user. Alternatively, the sub-region may be automatically selected. In some embodiments, it may comprise the whole or a pre-defined portion of the overlap region.

In some embodiments, each volumetric dataset corresponds to a cone-beam CT acquisition.

In some embodiments, each of the first and second images are 2D images.

In some embodiments, the method comprises controlling the user interface to provide a graphical user interface at the display. The graphical user interface may include a graphical region selector tool permitting a user to indicate the user-defined region of the first image.

In some embodiments, the region selector tool comprises a pre-defined region shape template, and wherein the received user input comprises a user definition of a position and/or size of the region shape template relative to the first image. For example, this might take the form of a circle or square which is moveable by the user over the image, e.g. in the style of an observation glass or window, and wherein the sub-region bounded by an outer periphery of the moveable shape template is rendered as a representation of the sub-region as it appears in the first sub-volume and/or as it appears in the second sub-volume.

In some embodiments, the method comprises: generating a first version of the second image in which the sub-region is rendered based on only said first CT volumetric dataset; and generating a second version of the second image in which the sub-region is rendered based on only said second CT volumetric dataset.

In some embodiments, the method may comprise controlling the user interface to provide a user control permitting a user to selectively display the first version of the second image, the second version of the second image, or an overlay of the first and second versions of the second image.

In some embodiments, a plurality of volumetric datasets may represent a cardiac region of a patient. The cardiac region may include one or more coronary arteries. The at least one overlap region may contain at least a portion of at least one coronary artery.

In some embodiments, the method may further comprise controlling a CT imaging apparatus to acquire the plurality of CT volumetric datasets by implementing a sequential CT scanning protocol.

Another aspect of the invention is a computer program product comprising computer program code configured, when run on a processor, to cause the processor to perform a method in accordance with any preceding claim.

Another aspect of the invention is a processing device. The processing device may comprise one or more processors.

The processing device is configured to receive a plurality of CT volumetric datasets, each corresponding a different axial position along a sequential CT scanning protocol, wherein axially neighboring volumetric datasets partially overlap in the axial direction across a respective overlap volume.

The processing device may be configured to generate a first image from the plurality of CT volumetric datasets, the first image including at least a portion of at least one of the overlap volumes, the at least one overlap volume representing an overlap between a first and second of the CT volumetric datasets.

The processing device may be configured to render and display a representation of the first image using a user interface.

The processing device may be configured to receive an indication of a sub-region of the first image coinciding at least partially with said at least one overlap region.

The processing device may be configured to generate a second image from the plurality of volumetric datasets in which the sub-region is rendered based on only said first CT volumetric dataset, only said second CT volumetric dataset, or based on a transparency overlay of both the first and second CT volume datasets.

The processing device may be configured to render and display the second image using the user interface.

Another aspect of the invention is a system, comprising: the processing device as outline above or in accordance with any embodiment described in this disclosure or in accordance with any claim of this application. The system may further comprise a CT imaging apparatus operatively coupled with the processing device, wherein the processing device is arranged to receive the plurality of CT volumetric datasets from the CT imaging apparatus.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines steps of an example method in accordance with one or more embodiments of the invention;
Fig. 2 is a block diagram of an example processing device and system in accordance with one or more embodiments of the invention;
Fig. 3 schematically illustrates an example CT imaging apparatus;
Fig. 4 schematically illustrates a single cone beam projection;
Fig. 5 schematically illustrates a volumetric projection dataset for a single axial step of a sequential CT imaging protocol;
Fig. 6 and Fig. 7 show 3D illustrations of example reconstruction volumes spanned by volumetric projection datasets for a single axial step of a sequential CT imaging protocol;
Fig. 8 schematically illustrates a sequence of axial sub-volumes acquired as part of a sequential scanning protocol, wherein neighboring sub-volumes overlap axially;
Fig. 9 illustrates the regions of overlap between the neighboring sub-volumes of Fig. 8;
Fig. 10 illustrates overlap regions between reconstructed axial volumes;
Fig. 11 illustrates a step artefact at the boundary between two sub-volumes in a reconstructed image slice;
Fig. 12 illustrates the overlap region within the image of Fig. 11;
Fig. 13 illustrates an example sub-region overlapping with the overlap region of the first image of Fig. 11;
Figs. 14-15 illustrates a selector tool used by a user to select the sub-region and the rendering and display of a second image in which the sub-region is rendered using data from only one of the two overlapping sub-volumes; and
Figs. 16-17 schematically illustrate a step artefact affecting visualization of an artery.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an image generation and display method for use in CT imaging, for example cone beam sequential (step-and-shoot) imaging. The method utilizes the overlap between axially neighboring projection datasets acquired in a sequential imaging procedure to generate a first and second image representation of a region, where the first image is formed by stitching together neighboring image volumes aiming to form a continuous image representation of the anatomy, and the second image is formed as a modified version of the first, wherein a portion of the overlap region between two neighboring image volumes is rendered using data from a single one only of the two overlapping volumes. This allows for an anatomical structure which extends over the overlap region to be visualized more accurately, free from any potential artefacts occurring at the boundary between neighboring volumes in the rendered image.

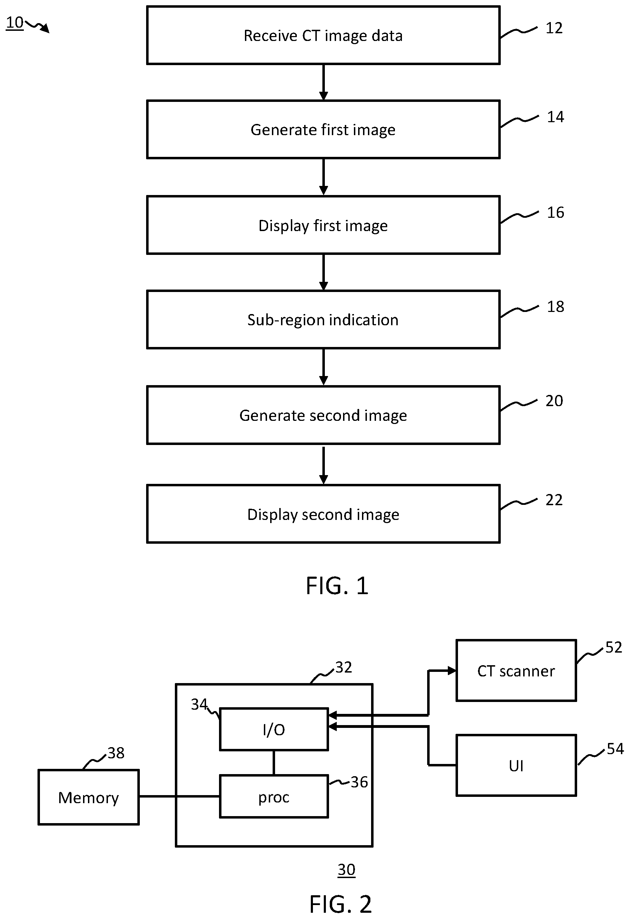

Fig. 1 outlines in block diagram form steps of an example method according to one or more embodiments. The steps will be recited in summary, before being explained further in the form of example embodiments.

The method 10 is for visualization of CT imaging data acquired in a sequential CT scanning protocol.

The method 10 comprises receiving 12 a plurality of CT volumetric datasets, each corresponding to an acquisition at a different axial position along a sequential CT scanning protocol, and wherein axially neighboring volumetric datasets partially overlap in the axial direction across a respective overlap volume. Each volumetric dataset for example comprises data spanning a respective sub-volume (slice) of an imaging zone of the scanner, and wherein the sub-volumes of axially neighboring volumetric datasets partially overlap in the axial direction across the respective overlap volume.

The method 10 further comprises generating 14 a first image of the imaging zone from the plurality of CT volumetric datasets, the first image including at least a portion of at least one of the overlap volumes, the at least one overlap volume representing an overlap between a first and second of the CT volumetric datasets. For example, the first image may be generated by stitching together the reconstructions of the plurality of volumetric datasets. The stitched image may include a transition region between each pair of axially neighboring volumetric datasets which are stitched together. The transition region may span all or part of the overlap region. The transition region may define a smoothed transition between a neighboring first and second volumetric dataset, or may define a hard boundary between a neighboring first and second volumetric dataset.

If the received volumetric datasets are projection datasets, the generating the first image may comprise reconstructing each of the projection volumetric datasets, to form a respective volumetric image dataset for each axial position, and then stitching these together. Alternatively, if the received volumetric datasets are image datasets, the method may comprise simply stitching the received volumetric datasets together.

The stitched image may be a volumetric image.

The first image may be a volumetric image or may be a 2D image rendered from the stitched 3D volume image.

The method 10 further comprises rendering and displaying 16 a representation of the first image using a user interface. This may comprise for example applying multi-planar reformatting (MPR) to the stitched volume image to render a 2D image view for display on the user interface spanning a plane lying within the stitched 3D volume. Alternatively, it may comprise applying volume rendering to render a 2D image view for display on the user interface from the stitched 3D volume.

The method 10 further comprises receiving an indication 18 of a sub-region of the first image coinciding at least partially with said at least one overlap region.

The method 10 further comprises generating 20 a second image of the imaging zone in which the sub-region is rendered based on only said first CT volumetric dataset, only said second CT volume dataset, or based on a transparency overlay of both the first and second CT volume datasets.

The method 10 further comprises rendering and displaying 22 the second image using the user interface.

For example, the second image may be generated by re-generating the stitched volumetric image, but wherein the transition zone between the first and second neighboring volumetric datasets is modified so that, within the sub-region, just image data from one of the two neighboring volumes is used. The second image may then be rendered from the stitched 3D volume for example using MPR or volume rendering.

For example, a first version and a second version of the stitched volumetric image may be re-generated: in the first version the sub-region is populated using data from only the first of the two neighboring volumetric datasets, in the second version the sub-region is populated using data from only the second of the two neighboring volumetric datasets.

In some embodiments, the user interface may permit selection of rendering and displaying a 2D plane from the first version or the second version of the image, or a transparency overlay of the two.

In accordance with at least a subset of embodiments, there is thus provided a system which enables a user to dynamically generate a new, different image from the sequence of axial volumes in which a particular region of the image spanning a transition zone between two of neighboring axial volumes is generated in a way that preserves the whole image information of at least one of the axial volumes. In other words, the new image is generated so that, within the sub-region, the appearance of the anatomy in that region as it appeared in at least one of the original axial volumes is visible to a viewer. This can be done by generating that region of the image using just image data from one of the axial volumes, or, by rendering a transparency overlay representation of the image data of both a first and second overlapping image volume across the user-indicated region.

As noted above, the method can also be embodied in hardware form, for example in the form of a processing device which is configured to carry out a method in accordance with any example or embodiment described in this document, or in accordance with any claim of this application.

To further aid understanding, Fig. 2 presents a schematic representation of an example processing device 32 configured to execute a method in accordance with one or more embodiments of the invention. The processing device is shown in the context of a system 30 which comprises the processing device. The processing device alone represents an aspect of the invention. The system 30 is another aspect of the invention. The provided system need not comprise all the illustrated hardware components; it may comprise only subsets of them.

The processing device 32 comprises one or more processors 36 configured to perform a method in accordance with that outlined above, or in accordance with any embodiment described in this document or any claim of this application. In the illustrated example, the processing device further comprises an input/output 34 or communication interface.

In the illustrated example of Fig. 2, the system 30 further comprises a CT imaging apparatus 52 for acquiring CT data. Details of the CT imaging apparatus 52 will be discussed in more detail below.

In the illustrated example of Fig. 2, the system 30 further comprises a user interface 52 which may comprise a display.

The system 30 may further comprise a memory 38 for storing computer program code (i.e. computer-executable code) which is configured for causing the one or more processors 36 of the processing unit 32 to perform the method as outlined above, or in accordance with any embodiment described in this disclosure, or in accordance with any claim.

As mentioned previously, the invention can also be embodied in software form. Thus, another aspect of the invention is a computer program product comprising computer program code configured, when run on a processor, to cause the processor to perform a method in accordance with any example or embodiment of the invention described in this document, or in accordance with any claim of this patent application.

In some embodiments, in the aforementioned first (stitched) image, the first and second datasets may be blended across the overlap region to form a composite image representation of the overlap region.

In some embodiments, in the aforementioned second image, the voxels of the first and/or second image datasets are preserved in the final image.

In some embodiments, each of the first and second images are 2D images.

In some embodiments, each received volumetric dataset corresponds to a cone-beam CT acquisition (at a different respective axial position along a sequential CT scanning protocol).

In some embodiments, in the first image, the at least one depicted overlap region includes a linear boundary between data of the first and second volume datasets overlapping at the overlap region.

In some embodiments, the first image and/or the second image is a non-axial 2D image plane.

In some embodiments, the first image and/or the second image spans at least two of the axial volumetric data sets.

As mentioned above, in some embodiments, the method comprises generating a first composite 3D image by axial stitching of the plurality of axial image volumes, and wherein the first image is rendered from the first composite 3D image.

In some embodiments, the axial stitching performed for the first volumetric image may comprise a first axial stitching operation which comprises a first compositing protocol for the overlap regions.

In some embodiments, the method may comprise applying image reconstruction to each axial volume dataset to convert each axial volume dataset from a projection dataset to an axial volume image.

The first compositing protocol for the overlap regions may comprise defining a transition zone between neighboring and overlapping axial volumes. In some embodiments this may include a boundary line at a location axially part way along the overlap region (e.g. halfway along), dividing the overlap region into a first part and a second part, and generating the first part of the overlap region using only data from the first image volume and generating the second part of the overlap region using only data from the second axial image volume. In other embodiments, the transition zone might be a smoothed transition zone.

In some embodiments, the first image may be a 2D image and may be generated by MPR applied to the first composite 3D image.

In some embodiments, generating the second image may comprise: generating a second composite 3D image, the second composite 3D image formed from the plurality of axial volume datasets, wherein the region of the second composite 3D image corresponding to the user-indicated region is generated differently than for the first composite 3D image, using only the first CT volume dataset, only the second CT volume dataset, or based on an overlay of both the first and second CT volume datasets.

In some embodiments, the second composite 3D image may be generated by performing a modification to the first composite 3D image in which the voxels coinciding with the user-indicated region are replaced with voxels determined using only the first CT volume dataset, only the second CT volume dataset, or based on an overlay of both the first and second CT volume datasets.

In some embodiments, the generating the second image does not comprise generating a second composite 3D image, but comprises applying a modification to the first image in which image values located within the user-indicated region are replaced with replacement image values determined based on only the first CT volume dataset, only the second CT volume dataset, or based on an overlay of both the first and second CT volume datasets.

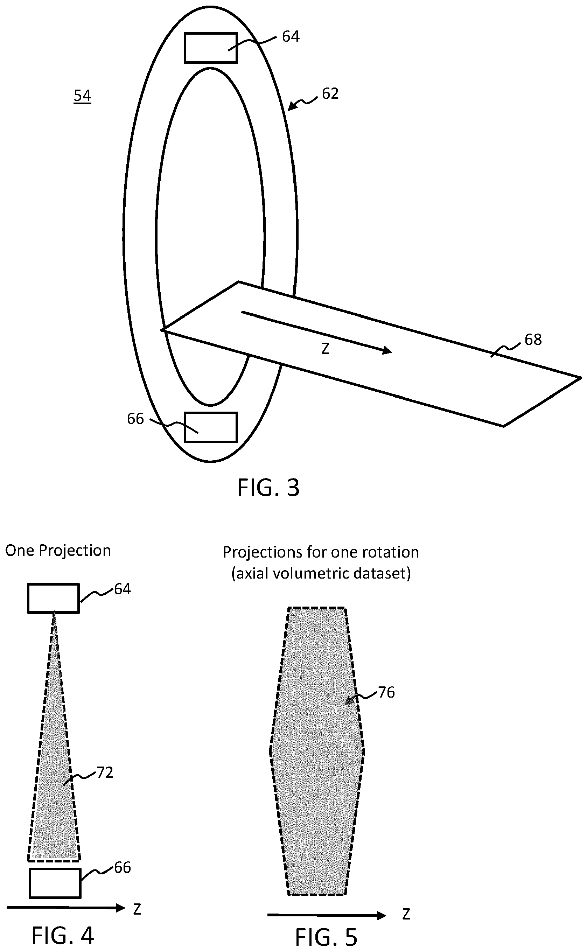

Fig. 3 shows a schematic representation of a CT imaging apparatus 54. The CT imaging apparatus comprises a rotatable gantry 62 housing an x-ray source 64 (e.g. an x-ray tube), and an x-ray detector 66. The x-ray detector may be a 2D x-ray detector. The CT imaging apparatus further comprises a patient table 68 which is moveable in an axial direction, z, relative to the gantry. With a patient disposed on the table 68, an x-ray beam emitted from the source 64 traverses an imaging zone around which the gantry extends, is attenuated by the tissue of the patient and is detected at the detector 66.

In a sequential cone beam CT acquisition method (step-and-shoot method), the patient or object remains stationary while the X-ray source 64 and detector 66 rotate around the patient to acquire data for a specific axial position. This results in a circular (or partial circular) trajectory cone beam scan. The system then moves (or "steps") to the next axial position, by changing a relative axial position between the table 68 and the gantry 62 (usually by moving the table) and another acquisition is performed. This sequence continues until all required axial positions are scanned.

Fig. 4 schematically illustrates a single cone beam projection 72 at a single rotation position of the source 64 and detector 66 system, and at a single axial, z, position of the scanner.

Fig. 5 schematically illustrates a cross-section or side view of complete set of cone beam volumetric projection data (for a whole rotation or part of a rotation) for a single axial position along a sequential scanning protocol. It can be seen that the set of data covers a disk-like volumetric region with cone-like projections at each end.

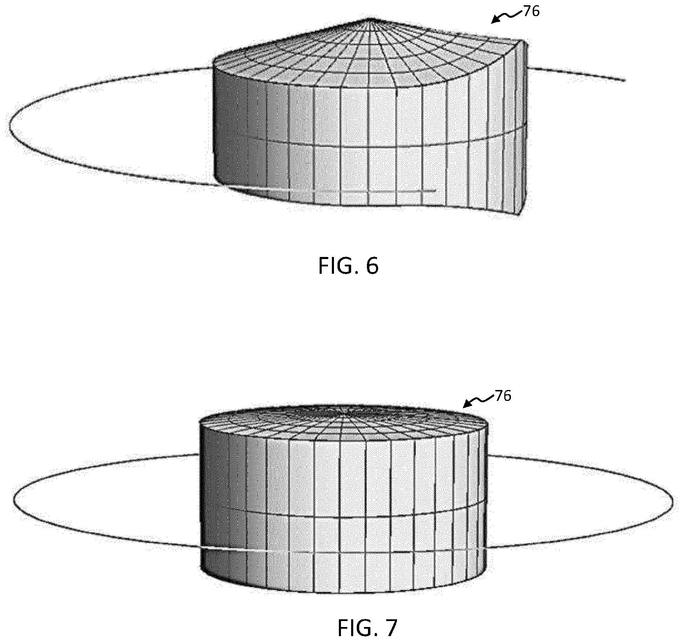

By way of further illustration, Fig. 6 and Fig. 7 each show a 3D illustration of an example volume region which may be reconstructed from a set of cone beam volumetric projection data. Fig. 6 shows a 3D illustration of the volume that can be reconstructed from an axial short scan (containing the volume that is within the cone over the entire short scan range). Fig. 7 shows a 3D illustration of the volume that can be reconstructed from an axial full scan (containing the volume that is within the cone over at least 180° after parallel rebinning). It is to be noted that, in both cases, the top surface is not flat, instead defining a cone-like projection region. The overlap between these cone-like projection regions of axially neighboring acquired volumes may at least partially form the previously discussed overlap region.

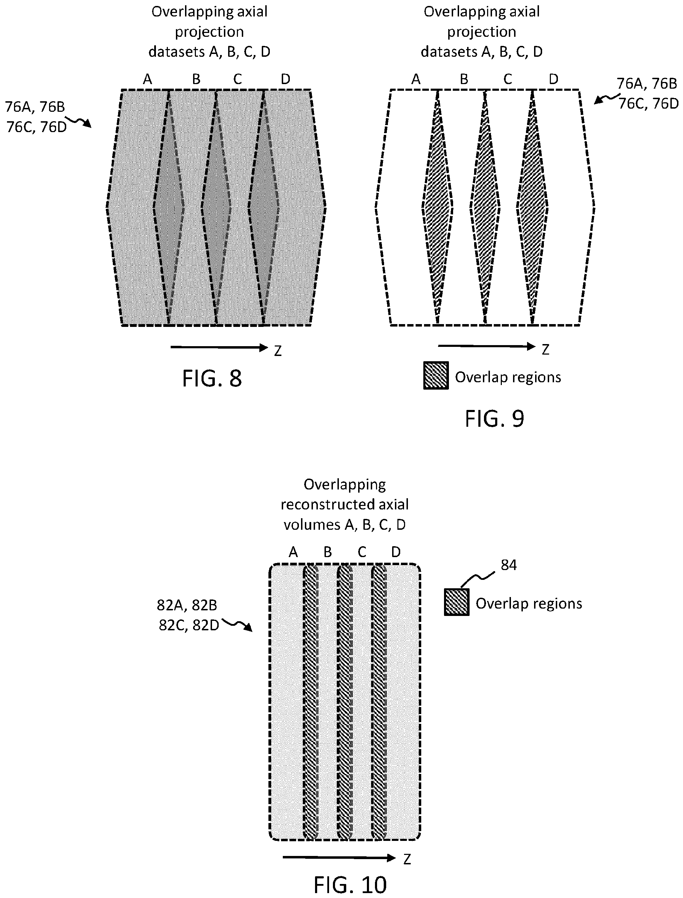

For example, Fig. 8 schematically illustrates an example sequence of axial volumetric datasets, each corresponding to a different axial location along a sequential CT imaging procedure. The four volumetric projection datasets are labelled 76A, 76B, 76C, 76D in Fig. 8.

The regions of overlap between the projection datasets are indicated in Fig. 9.

For each acquired axial volumetric dataset 76, a respective volumetric image may be reconstructed. This may for example take the form of a cylindrical volume image dataset (or near cylindrical, e.g. cylindrical with cone-like projections) for each axial position. For cone beam projection data, reconstruction of each axial volumetric image may be performed using an appropriate reconstruction algorithm, for example the Feldkamp-Davis-Kress (FDK) algorithm or other algorithm suitable for cone beam geometry. The result of the image reconstruction process is then a sequence of individual image volumes (e.g. cylindrical image volumes or cylindrical with approximately cone-shaped endings) corresponding to each axial position. Due to the overlap in the projection datasets, there is a resultant overlap in the axial, z, direction between axially neighboring volumetric images.

This is illustrated in Fig. 10 which shows schematically an example sequence of four reconstructed axial image volumes 82A, 82B, 82C, 82D, where regions of overlap 84 between neighboring volumes are illustrated.

The set of axial image volumes 82A, 82B, 82C, 82D are then composited into a unitary volumetric image representative of the complete imaging zone across which the sequence of axial acquisitions span.

The process of compositing the set of axial volumetric images 82 into a single image is sometimes known as 'stitching'.

The process of stitching may comprise a first step of registering axially neighboring axial image volumes to one another. Registration may be based on inherent features in the image (feature-based registration) or intensity values (intensity-based registration) to find an optimal transformation (translation, rotation, deformable, etc.) that aligns adjacent volumes.

Once the axial volumes are registered to one another, they may be stitched or fused together to create a single continuous volume. To achieve this, the overlap regions 84 between adjacent cylindrical volumes needs special consideration. There are several methods to handle the overlapping regions.

One simple approach is to have a linear boundary part way (axially) along the overlap region. To achieve this, one common approach is to select voxels from the volume based on the proximity of the position of the imaging source relative to the voxel in the axial direction. In particular, for each voxel in the overlapping region, the value from the volume with the source position closest to the z-position of the voxel is chosen.

To mitigate the impact of noise, an alternative approach is to average voxel values in the overlapping regions. By doing this, fluctuations in voxel values (often caused by noise) from the two overlapping volumes are reduced, leading to a smoother and potentially more accurate composite image. This approach, however, assumes that the two images being averaged are inherently consistent in the overlapping region (e.g. properly registered, and without major motion of the anatomy between acquisition of the two volumes).

Another common technique is weighted blending. In this method, rather than using an abrupt transition between two overlapping volumes, a smooth transition is created. The volumes are blended using a weighting factor that gradually changes from 1 to 0 towards the end of one volume in the z-direction, while inversely transitioning from 0 to 1 for the adjacent volume. This ensures that at the center of the overlapping region, both volumes contribute equally to the resultant composite image, while towards the edges, the contribution of one volume diminishes and the other dominates. Although this method can reduce visible artifacts in the overlap region, it can slightly compromise image sharpness due to the blending.

In some cases, combinations of these methods might also be used.

The final composite volume can be visualized using various image rendering techniques, including, by way of non-limiting example, multiplanar reformatting (MPR), maximum intensity projections (MIP), or 3D volume rendering.

Due to the fact that the final composite image volume is formed by stitching together axial image volumes acquired at different times, image artefacts can arise at the boundaries between stitched volumes, for example due to movement of the anatomy between the two acquisitions.

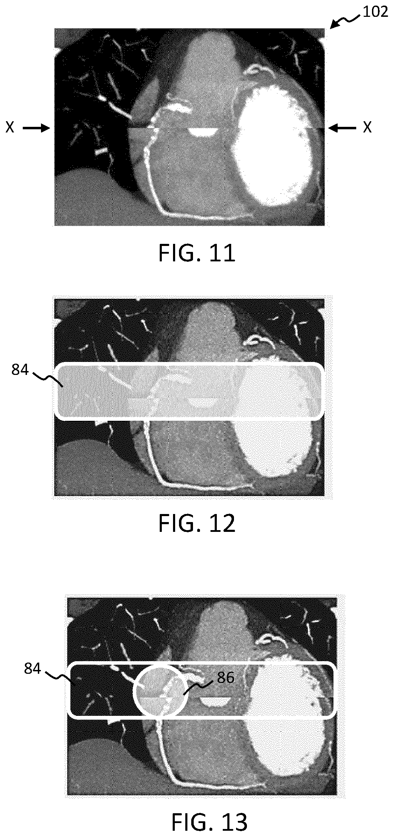

An example of a step artefact is illustrated in Fig. 11. Thus illustrates a first image 102 generated using image data of two neighboring axial volumetric images. The boundary between the two volumes is illustrated at line X. It may be observed that the coronary artery spans this boundary in the image. At the location of the boundary, there is a discontinuity in the representation of the artery due to the step artefact. This makes it difficult for a clinician to accurately interpret the artery in the image.

Embodiments of the present invention propose a method to assist in interpretation of images which may be vulnerable to such artefacts.

The proposed method comprises generating a second, modified, image of the same region depicted in the first image 102 in which at least a subregion 86 of the image which spans across the boundary, X, is generated using the image data of just a single one of the two neighboring axial volumes which meet at the boundary. This is possible because the boundary occurs at a location of the overlap region 84 across which the neighboring axial volumes overlap with one another. Accordingly, a subregion 86 within, or partially coinciding with the overlap region 84 can be rendered using image data from a selected one of the two neighboring volumes, or a transparency overlay of the two atop one another.

Fig. 12 schematically illustrates the overlap region 84 within the first image of Fig. 11. Fig. 13 illustrates selection of a sub-region 86 within the overlap region 84.

The boundary, X, may be a boundary line (defining a hard discontinuous boundary), or a boundary region, representing a transition region between the first and second volumes which are meeting at the boundary.

In other words, according to some embodiments, the method may comprise the following steps:
reconstructing each axial sub-volume to generate a respective sub-volume image for each axial position along the sequential CT scanning protocol;
generating a first image, wherein the generating the first image comprises stitching together the sub-volume images;
generating a second image, wherein the second image provides a same view of the imaged anatomy as the first image, but wherein a selected sub-region of the image overlapping with a boundary line or boundary region (e.g. transition region) between two neighboring axial volumes is rendered to show a continuous representation of:
   a portion of the first sub-volume which coincides with said sub-region, and/or
   a portion of the second sub-volume which coincides with said sub-region.

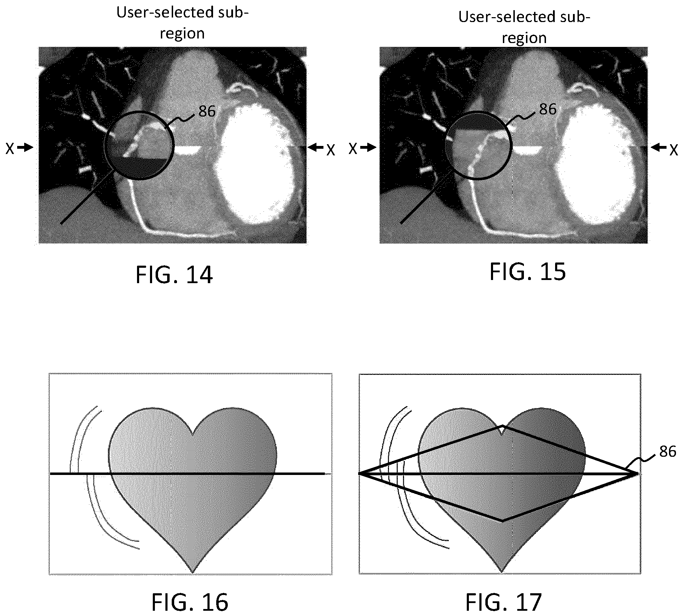

This is illustrated schematically in Fig. 14 and Fig. 15 which each show a user-selected sub-region 86, within which just the image data from one or the other of the two axial volumes meeting at the boundary, X, is shown. In Fig. 14, just image data from the upper of the two neighboring axial volumes is shown. In Fig. 15, just image data from the lower of the two neighboring axial volumes is shown.

In some embodiments, the user interface may be controlled to provide a user control permitting a user to selectively display one or more of: a first version of the second image in which the sub-region is rendered based on only said first CT volumetric dataset; a second version of the second image in which the sub-region is rendered based on only said second CT volumetric dataset; and an overlay of the first and second versions of the second image.

Thus, in summary, a method is proposed enabling display of an anatomical structure, e.g. a coronary artery, across a nominal border of two neighboring step and shoot axial volumes, thus offering an improved visualization of the anatomy, e.g. coronary artery and coronary artery lesions. It is based on the reconstruction of the complete image volume available in cone beam CT, e.g. circular cone beam CT. This leads to a volume overlap region between neighboring axial acquisitions which can be reconstructed from each of the two. Hence, a coronary artery crossing the border from the last slice of the first volume to the first slice of the adjacent volume can be displayed in two states for a certain number of slices. This effect can be used to increase the user's confidence in the assessment of coronary artery lesions located at the border of two sub-volumes.

In at least some embodiments the method may include the following steps.
- Reconstruction of all axial sub-volumes of a cardiac CT with the maximum achievable volume (including conical areas on top of the cylindrical reconstruction volumes).
- Stitching of the axial sub-volumes to yield a reconstructed 3D image spanning multiple of the axial sub-volumes.
- Rendering and displaying a first 2D image view across an area which spans a boundary between two neighboring axial sub-volumes. This represents the cardiac volume in its normal representation (adjacent cylindrical volumes as they have been reconstructed from the step and shoot acquisition).
- Display of a portion of the anatomy, e.g. a coronary artery, which crosses the border (region) between two sub-volumes using the first of the two neighboring sub-volumes, the second of the two neighboring sub-volumes, or an overlayed combination of both. :
   One display option is use of a type of `magic glass', wherein when the user clicks once on the anatomy portion (e.g. coronary artery) at the volume boundary, the anatomy portion is displayed from the first sub-volume. If the user clicks again, it is displayed from the second sub-volume, which at least partially overlaps with the first sub-volume.

This option could be extended by overlaying the displayed sub-volume with the anatomy outline (e.g. vessel contour) from the other sub-volume. If two different colors are used to indicate which sub-volume is currently displayed inside the magic glass, the overlayed vessel contour can be color-coded such that the color indicates from which sub-volume the contour is extracted.

Fig, 16 and Fig. 17 schematically illustrates a conical overlap region 86 between two axially adjacent reconstructed sub-volumes spanning over a cardiac region. A coronary artery spans across a boundary between the two sub-regions. A step-artefact is present meaning that there is a discontinuity in the representation of the artery at the boundary. Fig. 17 schematically shows a second image reconstruction in which the coronary artery contour as represented over the full overlap region for both the first and second overlapping volumes is depicted. This allows a user to view a representation of the coronary artery that is not interrupted at the boundary between the two reconstructed image sub-volumes.

Further optional features compatible with any of the embodiments described in this disclosure will now be discussed.

In accordance with some embodiments, there may be generated a plurality of second images, each of the plurality of second images generated from the plurality of CT volumetric datasets in which the sub-region is rendered based on only said first CT volumetric dataset, only said second CT volumetric dataset, or based on a transparency overlay of both the first and second CT volume datasets. In some embodiments, each of the plurality of second images may comprise a representation of the same axial acquisition position, and the same overlap region, but corresponding to a different respective time point along a cardiac cycle of the patient. To explain, in an axial or sequential CT scanning protocol, it is possible to acquire volumetric image data for each axial position which includes image data for more than one time point or time window. In particular, the minimum data required to reconstruct a single axial image volume is data acquired over a 180 degree rotation of the CT generator and detector. If data is acquired over more than a 180 degree rotation, multiple volumes can be reconstructed for the same axial position but corresponding to different time points because the scanner is effectively oversampling the region by covering it multiple times in quick succession. For example, a state of the art scanner may be able to complete a full rotation in significantly less than half a second, so that the data covered by half a rotation may cover just a portion of a heart cycle.

Thus, in some embodiments, each set of axial scan data (each CT volumetric dataset) may be acquired with a scan spanning more than approximately 180 degrees around the rotating gantry, such that image volumes for the same axial position but different points or portions of the heart cycle are obtained. It is possible then to reconstruct each axial volume and the overlap region at multiple cardiac phase points / time points. It is possible in this way to generate respective second images of a given anatomy region, including at least one overlap region, corresponding to multiple points or portions of the heart cycle (e.g., at 55%, 60%, 65%, 70%, 75%, 80% of the duration of the cardiac cycle). These could be displayed as separate images, and/or could be presented as a video image, e.g. a cineimage or cineloop, of the different time points. This would then show the overlap region across a number of time points or portions of the heart cycle.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A method (10) for visualization of CT scan data acquired in a sequential CT scanning protocol, the method comprising:
receiving (12) a plurality of CT volumetric datasets, each corresponding to a different axial acquisition position along the sequential CT scanning protocol, wherein axially neighboring volumetric datasets partially overlap in the axial direction across a respective overlap volume;
generating (14) a first image from the plurality of CT volumetric datasets, the first image including at least a portion of at least one of the overlap volumes, the at least one overlap volume representing an overlap between a first and a second of the CT volumetric datasets;
rendering and displaying (16) a representation of the first image using a user interface;
receiving (18) an indication of a sub-region of the first image coinciding at least partially with said at least one overlap region;
generating (20) a second image from the plurality of CT volumetric datasets in which the sub-region is rendered based on only said first CT volumetric dataset, only said second CT volumetric dataset, or based on a transparency overlay of both the first and second CT volume datasets;
rendering and displaying (22) the second image using the user interface.

2. The method of claim 1, wherein the method comprises controlling the user interface to present a user control permitting a user to select between display of the first image and display of the second image, e.g. to toggle between the first image and the second image.

3. The method of claim 1 or 2, wherein the receiving the indication of the sub-region comprises receiving a user input from the user interface indicative of a user-indicated region in the first image coinciding at least partially with said at least one overlap region.

4. The method of any preceding claim, wherein each volumetric dataset corresponds to a cone-beam CT acquisition.

5. The method of any preceding claim, wherein each of the first and second images are 2D images.

6. The method of any preceding claim,
wherein the method comprises controlling the user interface to provide a graphical user interface at the display;
wherein the graphical user interface includes a graphical region selector tool permitting a user to indicate the user-defined region of the first image.

7. The method of claim 6, wherein the region selector tool comprises a pre-defined region shape template, and wherein the received user input comprises a user definition of a position and/or size of the region shape template relative to the first image.

8. The method of any preceding claim, wherein the method comprises:
generating a first version of the second image in which the sub-region is rendered based on only said first CT volumetric dataset;
generating a second version of the second image in which the sub-region is rendered based on only said second CT volumetric dataset; and
controlling the user interface to provide a user control permitting a user to selectively display the first version of the second image, the second version of the second image or an overlay of the first and second versions of the second image.

9. The method of any preceding claim, wherein the plurality of volumetric datasets represent a cardiac region of a patient.

10. The method of claim 9, wherein the cardiac region includes one or more coronary arteries.

11. The method of claim 10, wherein the at least one overlap region contains at least a portion of at least one coronary artery.

12. The method of any preceding claim, further comprising controlling a CT imaging apparatus to acquire the plurality of CT volumetric datasets by implementing a sequential CT scanning protocol.

13. A computer program product comprising computer program code configured, when run on a processor, to cause the processor to perform a method in accordance with any preceding claim.

14. A processing device (32) configured to:
receive a plurality of CT volumetric datasets, each corresponding a different axial position along a sequential CT scanning protocol, wherein axially neighboring volumetric datasets partially overlap in the axial direction across a respective overlap volume;
generate a first image from the plurality of CT volumetric datasets, the first image including at least a portion of at least one of the overlap volumes, the at least one overlap volume representing an overlap between a first and a second of the CT volumetric datasets;
render and displaying a representation of the first image using a user interface (54);
receive an indication of a sub-region of the first image coinciding at least partially with said at least one overlap region;
generate a second image from the plurality of volumetric datasets in which the sub-region is rendered based on only said first CT volumetric dataset, only said second CT volumetric dataset, or based on a transparency overlay of both the first and second CT volume datasets;
render and display the second image using the user interface.

15. A system (30), comprising:
the processing device (32) of claim 14;
a CT imaging apparatus (52) operatively coupled with the processing device (32),
wherein the processing device is arranged to receive the plurality of CT volumetric datasets from the CT imaging apparatus.
